# EUROPEAN PATENT APPLICATION

(11) **EP 4 248 899 A1**
(43) Date of publication of application: **27.09.2023**
(21) Application number: 22305352.1
(22) Date of filing: 23.03.2022
(51) Int. Cl.: A61B 34/30, A61B 34/20, A61B 90/00, A61B 18/00

(54) **SURGICAL SYSTEM**

(71) Applicant: MinMaxMedical, 38400 Saint-Martin-d'Hères (FR)
(72) Inventor: DEHAN, Christophe, 38400 SAINT MARTIN D'HERES (FR)
(74) Representative: Regimbeau

(57) **Abstract**

The present invention concerns a surgical system (100), comprising at least
• one control unit (102) adapted to communicate with at least one storage unit (103),
• a robotic arm (110) with at least three motorized joints (114), the control unit (102) being adapted to determine, in real-time, the pose of each motorized joint (114),
• a passive arm (120) attached to a flange (112) of the robotic arm (110), comprising at least three distinct parallel axes (A1, A2, A3) forming passive joints (121), each passive joint (121) being equipped with one encoder adapted to determine, in real-time, an angular position of the concerned joint (121),
• a surgical device (130) adapted to treat a region of interest (140) of an anatomical structure according to a predefined surgical plane (P),
• a localization unit (150) comprising at least a first tracker (151) attached to a segment (113) or to a base (101) of the robotic arm (110) and at least a second tracker (152) adapted to be attached to the region of interest (140), the localization unit (150) being adapted to determine, in real-time, a relative pose of the segment (113) or the base (101) of the robotic arm (110) to which the first tracker (151) is attached with respect to the region of interest (140), the control unit (102) being adapted to
▪ determine a relative pose of the flange (112) of the robotic arm (110) with respect to the region of interest (140) based on the information provided by the localization unit (150) and/or on the pose of the motorized joints (114), and/or on stored geometry of the robotic arm (110),
▪ determine a relative pose of the surgical device (130) with respect to the surgical plane (P), based on the determined pose of the flange (112) of the robotic arm (110) with respect to the region of interest (140), on the angular positions of the passive arm's joints (121), on the stored pose of the surgical plane (P) with respect to the region of interest (140), and on stored geometries of the passive arm (120) and of the surgical device (130).

## Description

The present invention pertains to the domain of the surgical systems. Especially, the present invention concerns passive surgical systems adapted to be used while performing orthopedic surgeries.

The state of the art prior to robotic assistance, as the use of bone attached templates and guides to guide hand-held saw or other tools, limits resulting accuracy to surgeons' dexterity, in particular due to inaccuracy in guide placement and stability over patient anatomy and mandatory free mechanical play between tool and guide. Guide placement is intrusive as it also requires large access opening and fixation over bony structure. Using a robot to assist in cutting a plane in a bone with a cutting instrument such as a surgical saw, is now a well-known solution in orthopedic surgery. Different solutions have been proposed over the last few decades aimed to provide better accuracy, lower invasiveness, and lower complication rates, all contributing to expected better patient outcomes.

In a first approach, such as the one developed in the robot Rosa^{®} of the company Zimmer-Biomet, the robot carries a cutting guide, aligned with the plane to be cut. The surgeon thus has to insert the cutting instrument through the cutting guide to perform the planned cut. The robot here is adapted to maintain the cutting guide aligned with the plane to be cut. At least one drawback of such system is that it adds inaccuracy as it does not permit to take into account the play and bending of the cutting instrument in the cutting guide.

In a second approach, such as the robot Velys^{®} developed by Depuy Synthes, the robot carries a planar mechanism to which the cutting instrument is attached. The robot is here used to maintain the cutting instrument within the predefined plane to ensure that the surgeon who operates the cutting instrument follows the planned cut. In this second approach, the deviations of the saw from the planned plane are compensated thanks to an optical localization unit encompassing at least one tracker attached to the cutting instrument and at least a second tracker attached to the bone to be cut. At least one drawback of this kind of system is that the tracker attached to the cutting instrument must remain visible at any time for a camera of the localization unit which can be challenging considering the multiple angled positions that the cutting instrument must be put on during the cut(s). The presence of surgeon and helping hands around the surgery zone also typically results in frequent loss of line of sight between optical localization camera and tracker. The tracker also hampers the view of the surgery zone for the surgery personnel. Additionally, as the cutting instrument is, by definition, brought close to the anatomical structure treated, it can be soiled by blood or tissue projections. As well known in the art, a soiled tracker can result in a wrong evaluation of the pose of the associated object which adds inaccuracy or can be dangerous during such a surgical procedure.

Thus, it remains a need for an improved system which can determine and/or maintain, in real-time, the precise pose of the cutting tool with respect to the planned cutting plane. The present invention meets this objective whilst letting the surgeon freely move the saw or tool to remove bony material within that plane, combining robot's high accuracy and surgeon's expertise. Some optical localization line of sight issues, tracker close exposure to biologic projections and surgeon's cleared limited view of the surgery zone are also improved by the invention.

An object of the present invention thus concerns a surgical system, comprising at least
- one control unit adapted to communicate with at least one storage unit,
- a robotic arm with at least three motorized joints, the control unit being adapted to determine, in real-time the pose of each motorized joint,
- a passive arm attached to a flange of the robotic arm, the passive arm comprising at least three distinct parallel axes forming passive joints, each passive joint being equipped with one encoder adapted to determine, in real-time, an angular position of the concerned joint and to send a corresponding information to the control unit, the passive arm extending within a plane,
- a surgical device adapted to treat a region of interest of an anatomical structure according to a predefined surgical plane, the relative pose of the surgical plane with respect to the region of interest being stored in the storage unit and the surgical device being attached to the passive arm so that it extends according to a plane parallel to the passive arm plane,
- a localization unit comprising at least a first tracker attached to a segment of the robotic arm or to a base of this robotic arm, and at least a second tracker adapted to be attached to the region of interest, the localization unit being adapted to determine, in real-time, a relative pose of the segment or the base of the robotic arm to which the first tracker is attached, with respect to the region of interest, and to send a corresponding information to the control unit,
the control unit being adapted to
■ determine a relative pose of the flange of the robotic arm with respect to the region of interest based on the information provided by the localization unit and/or on the pose of the motorized joints and/or on a stored geometry of the robotic arm,
■ determine a relative pose of the surgical device with respect to the surgical plane, based on the determined pose of the flange of the robotic arm with respect to the region of interest, on the angular positions of the passive arm's joints, on the stored pose of the surgical plane with respect to the region of interest, and on stored geometries of the passive arm and of the surgical device.

As the robotic arm comprises at least three motorized joints, it also comprises several segments connected to one another thanks to these motorized joints. Thus, each segment of the robotic arm is delimited by two adjacent motorized joints.

The control unit is adapted to determine, in real-time, the poses of each motorized joint, for instance thanks to encoders housed within said motorized joint, or based on a pose of each motor driving said motorized joints. Obviously, any other known method could be implemented within the scope of the invention.

By "stored geometries" we here mean that the storage unit stores fixed and conformable dimensions and relations - such as by joint motion - between each part of the robotic arm with respect to one another, between each part of the passive arm with respect to one another between the parts of the robotic arm and of the passive arm which are connected to one another and also between part(s) of the passive arm and part(s) of the surgical device which are connected to each other. This definition applies for the whole document, adapted to the concerned objects.

The invention may be complemented by one or several of the following features, alone or in combination.

The control unit can be further adapted to
- determine if an active portion of the surgical device is within the surgical plane,
- compute at least one instruction to be sent to at least one of the motorized joints based on the determined relative pose of the surgical device with respect to the surgical plane, when the active portion of the surgical device is outside the surgical plane,
- send the computed instruction(s) to at least one of the motorized joints, the execution of such instruction(s) resulting in that the active portion of the surgical device is brought back within the surgical plane.

The surgical system comprises at least one display device, the control unit being adapted to compute a representation of the real-time poses of the region of interest and of the surgical device and to send an instruction to the display device, the execution of such instruction resulting in the display of said representation. Optionally, the control unit can be adapted to update the representation of the region of interest as the surgical device treats said region of interest.

The display device can be mounted on a base of the surgical system from which extends the robotic arm.

The display device can be mounted on an arm attached to the robotic arm.

The display device can be an augmented reality device adapted to be interposed between the region of interest and eyes of a user of the system.

According to an aspect of the invention, the surgical system comprises at least one sensor adapted to determine, in real-time, strain and/or forces and/or torques applied to the robotic arm and/or to the passive arm, and to send a corresponding information to the control unit. Advantageously, this information can include a quantification of the strains, forces and/or torques applied to the robotic arm and/or to the passive arm.

The control unit can be further configured to:
- determine a deformation of the robotic arm and/or of the passive arm, based on the determined strains and/or forces and/or torques applied to the robotic arm and/or to the passive arm, on a stored model of deformation of the robotic arm and/or of the passive arm, on the angular positions of the passive arm's joints and on stored geometries of the passive arm and of the surgical device,
- adjust the determined relative pose of the surgical device with respect to the surgical plane based on the determined deformation.

Advantageously, the control unit can be further adapted to:
- determine if an active portion of the surgical device is within the surgical plane,
- compute at least one instruction to be sent to at least one of the motorized joints based on the adjusted relative pose of the surgical device with respect to the surgical plane and on the computed deformation of the robotic arm and/or of the passive arm, when the surgical device is outside the surgical plane,
- send the computed instruction(s) to at least one of the motorized joints, the execution of such instruction(s) resulting in that the surgical device is brought back within the surgical plane.

The control unit can also be further configured to:
- receive an information relative to a quantification of strain and/or forces and/or torques applied to the robotic arm and/or to the passive arm,
- compare the received information with a first predefined threshold,
- send sensory feedback to the user when the received information exceeds the first predefined threshold.

The control unit can be further configured to:
- receive an information relative to a quantification of strain, and/or forces and/or torques applied to the robotic arm and/or to the passive arm,
- compare the received information with a second predefined threshold,
- send an instruction to stop the surgical device when the received information exceeds the second predefined threshold.

It is understood that the information relative to the quantification of strains, forces and/or torques applied to the robotic arm and/or to the passive arm is provided by the at least one sensor evoked above.

According to the invention, the at least one sensor comprises one or several of the following:
- a force sensor,
- a torque sensor,
- strain gauge,
- piezoelectric or piezo-resistive gauges,
- optic fiber strain measurement device.

The at least one sensor can be arranged at one or several of the following positions:
- between the flange of the robotic arm and the passive arm,
- between the passive arm and the surgical device,
- on at least one link of the passive arm, such link being delimited by two adjacent passive joints,

Alternatively or additionally, the at least one sensor can comprise at least two relative displacement sensors, arranged on either side of each passive joint of the passive arm. At least one of the passive joints is equipped with at least one brake. The at least one brake can be an electromagnetic brake.

According to an aspect of the invention, the control unit can be further adapted to:
- receive an information relative to a quantification of strain, and/or forces and/or torques applied to the robotic arm and/or to the passive arm,
- compare the received information with a third predefined threshold,
- send an instruction to activate the at least one brake when the received information exceeds the third predefined threshold.

The encoders of the passive arm are absolute encoders. According to this feature, the stored geometry of the passive arm is recorded with the corresponding values given by the encoders for each recorded geometry.

The surgical device comprises a cutting tool adapted to cut bony structures. The surgical device comprises a saw, a burr, a drill, a laser, a water jet, a scalpel, focused ultrasounds or a shaver. The surgical device is removable from the passive arm and can be replaced by a linear guide adapted to be attached to the passive arm, the linear guide being adapted to guide a cutting tool.

Further details and features of the invention are described below with reference to the following drawing:
- Figure 1 is a general perspective view of a surgical system according to an embodiment of the present invention;
- Figure 2 is an enlarged view of a surgical device of the surgical system treating a region of interest according to a surgical plane;
- Figure 3a and 3b illustrate two different examples of sensors positions included to the surgical system.

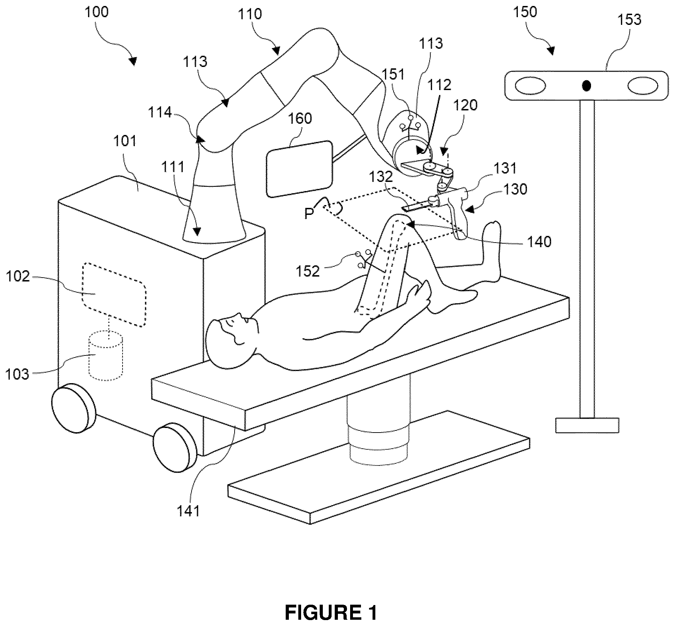

**Figure** 1 is a general view of a surgical system 100 according to the invention. The surgical system 100 comprises a base 101 from which extends a robotic arm 110 connected to a passive arm 120. For instance, the base 101 can be a wheeled cart. As shown, a surgical device 130 is attached to the passive arm 120. As detailed below, the surgical device 130 is adapted to treat a region of interest 140 according to a surgical plane P. According to the illustrated embodiment, the surgical device 130 comprises at least one power tool 131 adapted to power a cutting tool 132. By "cutting tool" we here mean that the tool is adapted to cut bony structures. For instance, this cutting tool can be saw, a burr, a drill, a laser, a water jet, a scalpel, focused ultrasounds or a shaver. Advantageously, the surgical device can be removably attached to the passive arm 120, so that it can be easily replaced by a linear guide adapted to guide a cutting instrument.

The robotic arm 110 extends between a first end 111 connected to the base 101 and a second end 112 which forms a flange of the robotic arm 110 to which the passive arm 120 is connected. The robotic arm 110 comprises several segments 113 connected to one another thanks to motorized joints 114 - only one of each being referenced on figure 1. According to the invention, the robotic arm 110 comprises at least three motorized joints 114 and it could comprise more than three motorized joints, for instance six motorized joints within the scope of the invention. Obviously, the robotic arm could also comprise more than six motorized joints without departing from the scope of the invention.

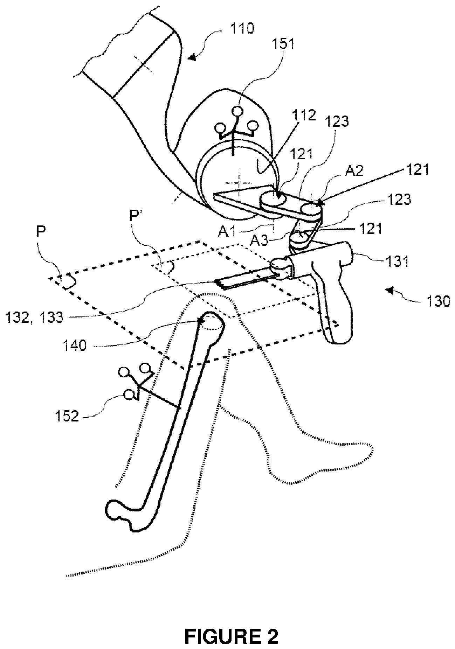

The passive arm 120 can be freely moved within a plane, hereafter referred to as the "passive arm plane". As shown on **figure 2****,** the surgical device 130 is attached to the passive arm 120 so that such surgical device 130 extends, mainly, within a plane P' parallel to the passive arm plane. The passive arm 120 comprises at least three axes A1, A2, A3 forming three distinct passive joints 121. The words "passive joints" are here used as opposed to "motorized joints" as they are manually displaceable and deprived of motors. Two adjacent passive joints 121 define one link 123 of the passive arm 120. Consequently, the passive arm 120 comprises at least two links 123.

According to an aspect of the invention, the robotic arm 110 is adapted to displace the passive arm 120 until the plane P' wherein the surgical device 130 mainly extends matches the surgical plane P. Especially, at least an active portion 133 of the surgical device 130 must be within the surgical plane P. By "active portion" we here mean the portion of the surgical tool which is adapted to actually cut the region of interest. According to the illustrated embodiment, the surgical device is formed as a surgical saw, the active portion being formed by a blade of such surgical saw. A user of the system can thus manipulate the surgical device 130 while ensuring that he/she is treating the region of interest 140 according to the predefined surgical plane P. As the surgical device 130 is maintained within the surgical plane P thanks to the robotic arm 110, the user of the system can operate the surgical device 130 with high precision and little effort. Thus, his/her sensitivity about the performed procedure is increased as the force feedback he/she receives is produced by the effort actually applied on the region of interest. Such a configuration is for instance illustrated on **figure 2****.**

The surgical system 100 also comprises at least one control unit 102 and at least one storage unit 103, the control unit 102 being adapted to communicate with the storage unit 103. This communication can be wired or wireless without departing from the scope of the invention. According to the illustrated embodiment, the control unit 102 and the storage unit 103 are encompassed within the base 101 of the system, but they could be positioned anywhere else within the system, or even be deported within the scope of the invention. The storage unit 103 is advantageously used to store one or several of the following:
- geometries of the robotic arm, of the passive arm and/or of the surgical device
- a relative pose of the surgical plane with respect to the region of interest
- a model of deformation of the robotic arm and/or of the passive arm
- a model of deformation of at least one force or torque sensor.

By "geometries" we here mean that the storage unit stores fixed and conformable dimensions and relations - such as by joint motion - between each part of the robotic arm with respect to one another, between each part of the passive arm with respect to one another between the parts of the robotic arm and of the passive arm which are connected to one another and also between part(s) of the passive arm and part(s) of the surgical device which are connected to each other. This definition applies *mutatis mutandis* in the whole document.

The relative pose of the surgical plane P with respect to the region of interest 140 is determined during a planning procedure. Several well-known methods can be used to realize such planning and they are not developed in this document.

As known by the skilled man of the art, the model of deformation of the robotic arm and/or of the passive arm can be determine by construction, or it can be determined later by conducting calibration tests with different loads applied at the end of the concerned arm.

Moreover, the control unit 102 is adapted to determine, in real-time, a pose of each motorized joint of the robotic arm. For instance, encoders can be housed within said motorized joints, or the control unit 102 can be connected to motors driving the motorized joints, thus permitting such control unit 102 to know, at any time, the current pose of the motors and to determine, consequently, the pose of each motorized joint.

As shown, the surgical system 100 further comprises a localization unit 150. According to the illustrated embodiment, this localization unit 150 is an optical localization unit, but it could be of any other known technology, such as an electromagnetic localization unit for instance, without departing from the scope if the invention. The localization unit comprises at least a first tracker 151 rigidly fixed to the robotic arm 110, a second tracker 152 rigidly attached to the region of interest 140 and a camera system 153 adapted to detect the current pose of the trackers 151, 152 and to determine, based on said current poses of the trackers and on a known geometry between each tracker and the corresponding tracked objects, the relative pose of the robotic arm 110 with respect to the region of interest. The localization unit is further adapted to communicate with the control unit 102.

As schematically illustrated, the first tracker 151 can for instance be attached to the last segment 113 of the robotic arm 110, i.e., the segment forming the flange 112 to which the passive arm is connected. Alternatively, the first tracker 151 could be arranged on the second to last segment. According to another alternative, the first tracker 151 could be arranged on the base 101 of the surgical system 100 within the scope of the invention. The localization unit 150 is adapted to determine, in real-time, a relative pose of the segment or the base of the robotic arm 110 to which the first tracker 151 is attached, with respect to the region of interest 140, and to send a corresponding information to the control unit 102.

The passive arm 120 further comprises at least three encoders housed within the passive joints 121. According to an aspect of the invention, each passive joint 121 is equipped with one of the encoders. The encoders are adapted to determine, in real-time an angular position of the passive joint 121 to which it is associated, and to send a corresponding information to the control unit 102. According to an aspect of the invention, the encoders can be absolute encoders. If so, the storage unit 103 also stores the values of each encoder with respect to specific geometries of the passive arm. Optionally, at least one brake can be housed in at least one of the passive joints 121. For instance, this brake can be an electromagnetic brake.

Finally, the surgical system 100 comprises at least one display device 160 adapted to display a representation computed by the control unit 102 of the current poses of the robotic arm 110 and of the region of interest 140. Thus, the user of the system can appreciate, at any time the relative pose of said objects. Advantageously, the representation of the region of interest can be updated, by the control unit 102, as the surgical device treats said region of interest. For instance, parts of the region of interest which have already been treated, i.e., parts where the surgical device 130 has already been operated, can be represented with different colors or textures. This is achieved thanks to an indirect tracking of the surgical device with respect to the region of interest that will be explained with more details below. According to the illustrated embodiment, the display device 160 is form as a display arranged on arm attached to the robotic arm 110. Obviously, this is merely an example and the display could be positioned on another part of the surgical system, such as its base, within the scope of the invention. Alternatively, the display could also be attached to an operating table 141 where a patient lies. According to yet another alternative, the display device could be an augmented reality device adapted to be positioned between the eyes of the user and the region of interest, such as augmented reality glasses, goggles, or panel.

As evoked above, the surgical system of the invention is adapted to help a user performing a planar cut on the region of interest. For instance, such system can thus be used for performing an osteotomy, or to prepare a bone to receive an artificial implant for instance in the case of a total or partial knee arthroplasty, or of a hip arthroplasty or an ankle arthroplasty etc.... First of all, the control unit 102 is adapted to compute instruction(s) to be sent to at least one of the motorized joints, the execution of which resulting in a displacement of the robotic arm 110 so that the plane P' wherein the surgical device extends matches the surgical plane P. A user of the system can then operate the surgical device along the surgical plane P and perform the planned cut safely.

As the user manipulates the passive arm 120, for instance by grasping the surgical device 130, he/she can apply inadvertently, forces and/or torques on the passive arm 120 which could result in a deviation of the surgical device 130, and especially of the active portion 133 of such surgical device 130, which could then be positioned outside the surgical plane P.

The localization unit 150 monitors, continuously, the relative pose of the segment 113 or the base 101 of the robotic arm 110 to which the first tracker 151 is attached, with respect to the region of interest 140 and sends corresponding information to the control unit 102. Based on this information, and/or on the pose of the motorized joints and/or on the stored geometry of the robotic arm, the control unit 102 is adapted to determine a relative pose of the flange 112 of the robotic arm 110 with respect to the region of interest 140.

It is understood that the information needed to determine the relative pose of the flange 112 with respect to the region of interest 140 are different depending on the position of the first tracker 151. For instance, if the first tracker 151 is attached to the last segment 113 of the robotic arm 110, the information provided by the localization unit 150 alone is sufficient. But if the first tracker 151 is attached to the base 101 of the robotic arm 110, then the control unit 102 needs the information provided by the localization unit 150, combined with the pose of the motorized joints 114, combined with the stored geometry of the robotic arm 110 to determine the current pose of the flange 112 with respect to the region of interest 140.

Then, the control unit 102 is adapted to determine the relative pose of the surgical device 130 with respect to the surgical plane P, based the determined pose of the flange 112 with respect to the region of interest 140, on the angular positions of the passive arm's joint transmitted by the encoders, on the stored relative pose of the surgical plane P with respect to the region of interest 140 and on stored geometries of the passive arm 120 and of the surgical device 130.

The system 100 thus permits to track the surgical device 130 without needing to attach a tracker on such surgical device 130. As the geometry of the surgical device 130 is stored, the control unit 102 can determine the pose of the active portion 133 of such surgical device 130 with respect to the surgical plane P based on the determined pose of said surgical device 130.

As the system is adapted to ensure that the surgical device 130, and especially its active portion 133, remains within the surgical plane P, the control unit 102 is further adapted to compute at least one instruction and to send it/them to at least one of the motorized joints, the execution of this instruction(s) resulting in that the active portion of the surgical device 130 is brought back within the surgical plane P.

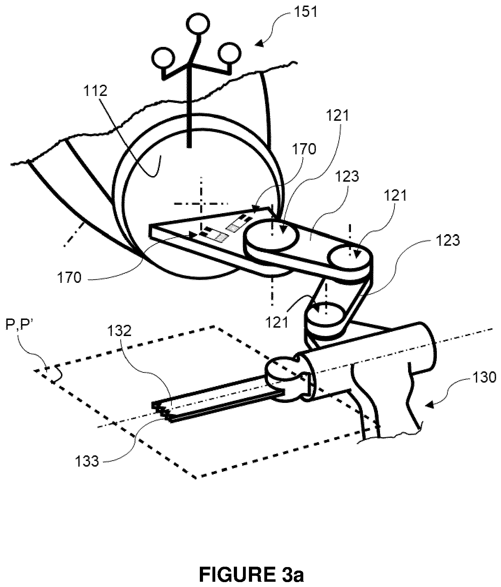

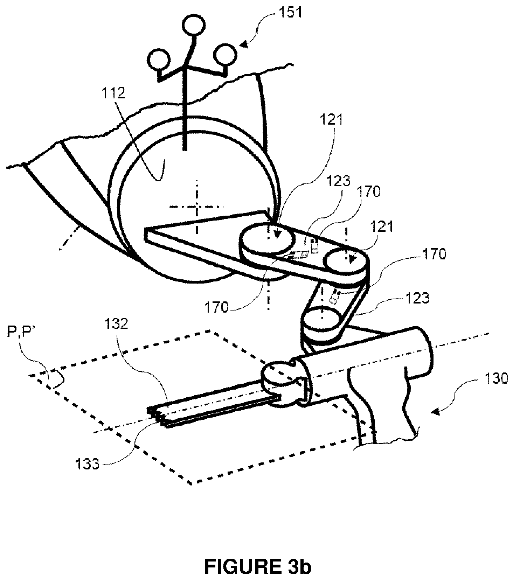

As for instance illustrated on **figures 3a****,** 3b, the surgical system 100 of the invention can comprise at least one sensor 170 adapted to determine strains and/or torques and/or forces applied on the robotic arm 110 and/or on the passive arm 120. Figures 3a and 3b differs from one another in the positioning of such sensors. Figure 3a illustrates a first example wherein the at least one sensor 170 comprises two strain gauges arranged between the flange 112 of the robotic arm 110 and the passive arm 120. Figure 3b illustrates a second example wherein the at least one sensor 170 comprises three strain gauges arranged on both links 123 of the passive arm 120. Especially, a first strain gauge is arranged on a last link 123 of the passive arm, i.e., the link to which is connected the surgical device 130 and two other strain gauges are arranged on a first link 123 of the passive arm, i.e., the link connected to the robotic arm 110.

It is understood that those strain gauges could be replaced by any other known strains, torques or forces sensor within the scope of the invention. Examples of strains, forces or torques sensors compatible with the present invention are given below. Also, the surgical system could comprise more or less sensors than illustrated without departing from the scope of the invention.

Obviously, those are only examples of how to carry the invention and the at least one sensor could be in another position with respect to the system within the scope of the invention. Also, the system could comprise several sensors or different kind at several locations without departing from the scope of the invention.

When the system is equipped with such sensor(s) 170, the control unit 102 is further adapted to determine a deformation of the robotic arm 110 and/or of the passive arm 120, based on the determined strains and/or forces and/or torques applied to the robotic arm and/or to the passive arm, on the angular positions of the passive joints transmitted by the encoders and on the stored model of deformation of the robotic arm and/or of the passive arm. The control unit 102 is then adapted to adjust the previously determined relative pose of the surgical device 130 with respect to the surgical plane P based on said determined deformation.

The control unit 102 can be further adapted to determine whether the surgical device 130, and especially its active portion 133, is within the surgical plane P or not. When the active portion 133 of the surgical device 130 is outside the surgical plane P, the control unit 102 is configured to compute at least one instruction to be sent to at least one of the motorized joints in order to bring back the active portion 133 of the surgical device 130 within the surgical plane P. This at least one instruction is computed based on the adjusted relative pose of the surgical device 130 with respect to the surgical plane P and on the computed deformation of the robotic arm and/or of the passive arm. The instruction(s) can then be sent to at least one of the motorized joints in order to bring the active portion 133 of the surgical device 130 back within the surgical plane P.

The control unit 102 can also be adapted to compare the strains and/or forces and/or torques applied to the robotic arm and to the passive arm with a predefined first threshold and to provide feedback to the user when said strains, forces and/or torques exceed said first threshold. This feedback can for instance be tactile feedback, visual feedback or auditive feedback. Obviously, any other sensorial feedback could be used without departing from the scope if the invention.

Additionally, the control unit 102 can be adapted to compare the strains, forces and/or torques applied to the robotic arm with a second threshold, greater than the first threshold, and to stop the surgical device to prevent any unwanted cut when the strains, forces and/or torques are above this second threshold. Additionally or alternatively, the control unit can be adapted to activate the brakes of the passive joints when the strains, forces and/or torques are above a third threshold, this third threshold being greater than the first threshold and than the second threshold.

According to different embodiments of the invention, the at least one sensor 170 can comprise one or several of the following:
- a force sensor,
- a torque sensor,
- strain gauge,
- piezoelectric or piezo-resistive gauges,
- optic fiber strain measurement device.

According to the invention, the at least one sensor 170 can be arranged at one or several of the following positions:
- between the flange 112 of the robotic arm 110 and the passive arm 120, as illustrated on figure 3a,
- between the passive arm 120 and the surgical device 130,
- on at least one link 123 of the passive arm 120, as illustrated on figure 3b.

According to an aspect of the invention, the at least one sensor 170 can additionally or alternatively comprise at least two relative displacement sensors, arranged on either side of each passive joint 121 of the passive arm 120. By monitoring the relative displacement of the links 123 connected by the concerned passive joint 121, the relative displacement sensors permit to detect deflection and torsion applied on either of said links.

Obviously, the surgical system 100 could comprise a combination of those without departing from the scope of the invention.

## Claims

1. A surgical system (100), comprising at least
• one control unit (102) adapted to communicate with at least one storage unit (103),
• a robotic arm (110) with at least three motorized joints (114), the control unit (102) being adapted to determine, in real-time, the pose of each motorized joint (114),
• a passive arm (120) attached to a flange (112) of the robotic arm (110), the passive arm (120) comprising at least three distinct parallel axes (A1, A2, A3) forming passive joints (121), each passive joint (121) being equipped with one encoder adapted to determine, in real-time, an angular position of the concerned joint (121) and to send a corresponding information to the control unit (102), the passive arm (120) extending within a plane,
• a surgical device (130) adapted to treat a region of interest (140) of an anatomical structure according to a predefined surgical plane (P), the relative pose of the surgical plane (P) with respect to the region of interest (140) being stored in the storage unit (103) and the surgical device (130) being attached to the passive arm (120) so that it extends according to a plane (P') parallel to the passive arm plane,
• a localization unit (150) comprising at least a first tracker (151) attached to a segment (113) of the robotic arm (110) or to a base (101) of this robotic arm (110) and at least a second tracker (152) adapted to be attached to the region of interest (140), the localization unit (150) being adapted to determine, in real-time, a relative pose of the segment (113) or the base (101) of the robotic arm (110) to which the first tracker (151) is attached with respect to the region of interest (140), and to send a corresponding information to the control unit (102),
the control unit (102) being adapted to
■ determine a relative pose of the flange (112) of the robotic arm (110) with respect to the region of interest (140) based on the information provided by the localization unit (150) and/or on the pose of the motorized joints (114), and/or on stored geometry of the robotic arm (110),
▪ determine a relative pose of the surgical device (130) with respect to the surgical plane (P), based on the determined pose of the flange (112) of the robotic arm (110) with respect to the region of interest (140), on the angular positions of the passive arm's joints (121), on the stored pose of the surgical plane (P) with respect to the region of interest (140), and on stored geometries of the passive arm (120) and of the surgical device (130).

2. The surgical system (100) according to claim 1, wherein the control unit (102) is further adapted to
• determine if an active portion (133) of the surgical device (130) is within the surgical plane (P),
• compute at least one instruction to be sent to at least one of the motorized joints (114) based on the determined relative pose of the surgical device (130) with respect to the surgical plane (P), when the active portion (133) of the surgical device (130) is outside the surgical plane (P),
• send the computed instruction(s) to at least one of the motorized joints (114), the execution of such instruction(s) resulting in that the active portion (133) of the surgical device (130) is brought back within the surgical plane (P).

3. The surgical system (100) according to any of the preceding claims, comprising at least one display device (160), the control unit (102) being adapted to compute a representation of the real-time poses of the region of interest (140) and of the surgical device (130) and to send an instruction to the display device (160), the execution of such instruction resulting in the display of said representation.

4. The surgical system (100) according to the preceding claim, wherein the control unit (102) is adapted to update the representation of the region of interest (140) as the surgical device (130) treats said region of interest (140).

5. The surgical system (100) according to any of claims 3 or 4, wherein the display (160) is an augmented reality device adapted to be interposed between the region of interest (140) and eyes of a user of the system (100).

6. The surgical system (100) according to any of the preceding claims, comprising at least one sensor (170) adapted to determine, in real-time, strain and/or forces and/or torques applied to the robotic arm (110) and/or to the passive arm (120), and to send a corresponding information to the control unit (102).

7. The surgical system (100) according to the preceding claim, wherein the control unit (102) is further configured to:
• determine a deformation of the robotic arm (110) and/or of the passive arm (120), based on the determined strain and/or forces and/or torques applied to the robotic arm (110) and/or to the passive arm (120), on stored model(s) of deformation of the robotic arm (110) and/or of the passive arm (120), on the angular positions of the passive arm's joints (121) and on stored geometries of the passive arm (120) and of the surgical device (130),
• adjust the determined relative pose of the surgical device (130) with respect to the surgical plane (P) based on the determined deformation.

8. The surgical system (100) according to the preceding claim, wherein the control unit (102) is further adapted to:
• determine if an active portion (133) of the surgical device (130) is within the surgical plane (P),
• compute at least one instruction to be sent to at least one of the motorized joints (114) based on the adjusted relative pose of the surgical device (130) with respect to the surgical plane (P) and on the computed deformation of the robotic arm (110) and/or of the passive arm (120), when the surgical device (130) is outside the surgical plane (P),
• send the computed instruction(s) to at least one of the motorized joints (114), the execution of such instruction(s) resulting in that the surgical device (130) is brought back within the surgical plane (P).

9. The surgical system (100) according to claim 6, wherein the control unit (102) is further configured to:
• receive an information relative to a quantification of strain and/or forces and/or torques applied to the robotic arm (110) and/or to the passive arm (120),
• compare the received information with a first predefined threshold,
• send sensory feedback to the user when the received information exceeds the first predefined threshold.

10. The surgical system (100) according to any of claims 6 or 9, wherein the control unit (102) is further configured to:
• receive an information relative to a quantification of strain, and/or forces and/or torques applied to the robotic arm (110) and/or to the passive arm (120),
• compare the received information with a second predefined threshold,
• send an instruction to stop the surgical device (130) when the received information exceeds the second predefined threshold.

11. The surgical system (100) according to any of claims 6 to 10, wherein the at least one sensor (170) comprises one or several of the following:
• a force sensor,
• a torque sensor,
• strain gauge,
• piezoelectric or piezo-resistive gauges,
• optic fiber strain measurement device.

12. The surgical system (100) according to of claims 6 to 11, wherein the at least one sensor (170) is arranged at one or several of the following positions:
• between the flange (112) of the robotic arm (110) and the passive arm (120),
• between the passive arm (120) and the surgical device (130)
• on at least one link (123) of the passive arm (120), such link (123) being delimited by two adjacent passive joints (121).

13. The surgical system (100) according to any of claims 6 to 12, wherein the at least one sensor (170) comprises at least two relative displacement sensors, arranged on either side of each passive joint (121) of the passive arm (120).

14. The surgical system (100) according to any of the preceding claims, wherein at least one of the passive joints (121) is equipped with at least one brake.

15. The surgical system (100) according to claim 6 in combination with claim 14, wherein the control unit (102) is further adapted to:
• receive an information relative to a quantification of strain, and/or forces and/or torques applied to the robotic arm (110) and/or to the passive arm (120),
• compare the received information with a third predefined threshold,
• send an instruction to activate the at least one brake when the received information exceeds the third predefined threshold.

16. The surgical system (100) according to any of the preceding claims, wherein the surgical device (130) comprises a cutting tool adapted to cut bony structures.

17. The surgical system (100) according to the preceding claim, wherein the surgical device (130) comprises a saw, a burr, a drill, a laser, a water jet, a scalpel, focused ultrasounds or a shaver.

18. The surgical system (100) according to any of claims 1 to 15, wherein the surgical device (130) is removable from the passive arm (120) and can be replaced by a linear guide adapted to be attached to the passive arm (120), the linear guide being adapted to guide a cutting tool.
